(19) Europäisches Patentamt
European Patent Office
Office européen des brevets

(11) **EP 4 582 077 A1**

(12) **EUROPEAN PATENT APPLICATION**

(43) Date of publication:
**09.07.2025 Bulletin 2025/28**

(21) Application number: **25150310.8**

(22) Date of filing: **06.01.2025**

(51) International Patent Classification (IPC):
***A61K 8/42*** *(2006.01)* ***A61Q 5/00*** *(2006.01)*
***A61Q 5/12*** *(2006.01)*

(52) Cooperative Patent Classification (CPC):
**A61Q 5/004; A61K 8/42; A61Q 5/12;**
A61K 2800/31; A61K 2800/34

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.01.2024 IN 202421001382**

(71) Applicant: **Galaxy Surfactants Ltd.**
**Navi Mumbai 400 703 Maharashtra (IN)**

(72) Inventors:
- **VAIDYA, Pooja**
**440022 Laxmi Nagar, Nagpur, Maharashtra (IN)**
- **ABHICHANDANI, Bharat Ghanshyamdas**
**413801 Daund, Pune, Maharashtra (IN)**
- **GHADIGAONKAR, Sneha Shailesh**
**400605 Parsik Nagar, Kalwa (West), Thane, Maharashtra (IN)**
- **VORA, Chintan Navin**
**400025 Prabhadevi, Mumbai, Maharashtra (IN)**

(74) Representative: **Müller Schupfner & Partner Patent- und Rechtsanwaltspartnerschaft mbB (Hamburg)**
**Schellerdamm 19**
**21079 Hamburg (DE)**

# (54) MILD, NATURAL, SOLID COMPOSITIONS FOR WATERLESS, SUSTAINABLE, SILICONE FREE COLOR AND UV PROTECTION FORMATS

(57) Provided herein is a mild, natural, sustainable, silicon-free, waterless, solid hair color protection and UV protection composition comprising of p-methoxy cinnamidopropyl behenyl dimethyl ammonium chloride, one or more structuring agent like fatty alcohol, natural conditioning agents like butters, natural vegetable/fruit/ nut oil and its corresponding esters. The pH of the solid hair color protection composition is 3.5 to 5.5.

EP 4 582 077 A1

**Description**

## FIELD OF INVENTION

The present invention relates to mild, natural, sustainable hair color and UV protection composition which can be further heated and molded to form homogenous, water less, hair color and UV protection bar and powder format. The bar or powder form when mixed with water at the time of use provides excellent ease of application on hair to the consumers.

## BACKGROUND OF INVENTION

Personal care industry has come a long way in past few decades. Shampoos are very common hair-care products used for hair cleansing. In addition to hair cleansing consumer expects shampoos to leave hair soft, smooth in a manageable condition. Shampoos are mainly composed of anionic surfactants which are known as efficient cleansing agent. In addition to anionic surfactants, amphoteric and non-ionic surfactants are also used in the formulations. Hence, shampoo cleanses hair effectively but leaves hair and scalp dry and causes certain level of damage. Therefore, formulators started using conditioning agents like cationic polymer and other cationic surfactants in shampoos. But cationic molecules are not very effective as they form complex in presence of anionic molecules. Hence, conditioning delivered via shampoo through coacervate formation is very limited. Therefore, separate conditioning formats came in to existence which are applied post shampooing hair and provide descent level of conditioning.

In addition to conditioning, consumer has lots of other expectations from the products such as aesthetics, mildness, ease of use, etc. Today's consumers are well educated and want to avoid the use of products harmful to human health as well as to the environment. This led to the need of natural and biodegradable formulations.

Various conditioning formats like liquid conditioners, conditioning masks etc. contain more than 70 % of water with maximum active content of around 30 %. Presence of high concentration of water helps in providing ease to use such formulations to the consumers but on the other hand leading to high carbon footprints during transportation. These diluted formulations are low viscous and therefore require proper packaging. More than 95% personal care formulations are packed in plastic bottles leading to the concern of plastic recycling or wastage.

Though water appears plentiful globally, it is known that only 3% of it is available as fresh water on earth, only 0.4% is available for usage as the rest is tied up in glaciers, snowpacks and ice caps. As per expert's opinion if significant responsible changes are not made in water consumption, two-third of the world may be facing water stress by the year 2025. Also, with regards to plastics, it is estimated that only 9 % of all plastics produced is recycled, the rest gets dumped in landfills and oceans resulting in pollution, causing harm to birds, marine, mammal and fish.

To overcome above mentioned issues, personal care industries are considering development of sustainable and solid formulations. WO2003057182A1 directs to use format like bars, powder and stick, reducing the usage of water. However, such formats have very less acceptance in the market as available formulations in the market does not provide ease of use, are not efficacious and are still not considered as convenient forms by consumers.

Also due to complexity of the formulations there are very few options of sustainable conditioning formats available in the market like Ethique and Earthkind conditioning bar which are limited to only some of the geographies available in Europe.

Coloring hair is another increasing global trend. The colors that are applied on hair, by nature of their chemical structures, hair are susceptible to the damaging effect of light and heat. This results in the appreciable degree of fading of the applied color. Curling, straightening, bleaching and coloring of hair causes damage substantially since these procedures involve intense heat and chemical treatment. Some of the procedures involve breaking down of covalent bonds by chemical treatments involving very harsh chemicals like sodium hydroxide or strong oxidizing agents like hydrogen peroxide. This kind of chemical assault coupled with damaging effect of solar radiation makes hair rough, brittle, dull and lifeless. The damage due to heat, chemicals and UV light to hair cuticles is easily felt and seen.

A close examination of products available in market reveals that there are some of the liquid rinse-off conditioners available for hair color and UV protection but surprisingly none of the conditioning bars available in the market is designed for hair protection from UV radiation and thereby hair color protection.

Mostly water-soluble UV absorbers such as benzophenone-4 (B-4) or phenyl benzimidazole sulphonic acid (PBSA) are used for hair color protection. Occasionally, one sees use of oil-soluble UV absorbers like octyl methoxy cinnamate in conjunction with water-soluble UV absorbers in hair care formulations that are targeted for color protection. However, via rinse-off applications the deposition of water-soluble UV absorber is too insignificant to protect hair or the applied hair color from the damaging effect of sunlight.

To overcome this challenge water-soluble cationic UV absorbers (US Patent 5,601,811) have been developed. Croda's Incroquat UV-283, cinnamido propyl trimethyl ammonium chloride is one such quaternary UV absorber.

Another US Patent 6,426,435 reports water-soluble quaternary UV absorbers, bis-quaternary salts of cinnamidoalkylamines. Though these are quaternary (cationic) molecules and do have higher substantivity/affinity to hair

tresses compared to nonquaternized UV absorbers, their extremely high water-solubility restricts the extent of deposition on hair through rinse-off of formulation.

Yet another US patent 6,531,628 teaches the use of water-soluble 2-hydorxy sulphobetaine moiety attached to UV absorbing chromophore. Whether the betaine type or the quaternary ammonium type, both classes exhibit better substantivity than simple salts of water-soluble sunscreen sulphonic acids (B-4 or PBSA), however, the extremely high water-solubility works against any meaningful deposition on hair surface. This difficulty, in part, was overcame by water-insoluble quaternized UV absorbers that have UV absorbing moiety attached to lauryl group (C-12) through a quaternary nitrogen center. The commercial products known as Escalol HP 610 by Ashland Chemicals and Galaxy TosyQuat by Galaxy Surfactants Ltd. US Patent 5,427,773 discloses cationic photo-filter based on dimethyl amino benzamidopropyl moiety (Escalol HP 610) whereas US Patent 6,613,340 discloses cationic filters based on cinnamido moiety (TosyQuat) and in both cases, the hydrophobicity has been imparted to the molecule by the long hydrocarbon chain of twelve carbon atoms that is attached to a quaternary nitrogen. By virtue of being hydrophobic due to long alkyl chain and tosylate as counter anion, these quaternized UV absorbers do exhibit good substantivity to hair tresses but they suffer from two major disadvantages, namely, difficult to formulate and difficult to synthesize and thereby becoming cost-ineffective for the benefits derived. Also, none of the above-mentioned options are suitable for waterless sustainable format.

The aforesaid limitations were overcome by Koshti et al (US 9,463,337 B2) which describes a novel process to manufacture water-dispersible, substantive, quaternary ammonium compound, *p*-methoxy cinnamidopropyl behenyl dimethyl ammonium chloride and its application as hair color protector in various personal care formulations but again the invention was limited to aqueous formulations only.

Some of the other commercially available actives are (1) CRODAFOS HCE (INCI: Oleth-5 Phosphate (and) Dioleyl Phosphate) (2) chromohance™ 113 (INCI: Polyquatemium-113 ) both the above mentioned molecules do not contain any UV active moiety for providing protection again harmful UV radiation; and (3) ABIL® UV Quat 50 (INCI: Polysilicone-15), it has cinnamic acid conjugate but available as a solution in dipropylene glycol hence make it difficult to formulate solid composition and also is not a silicone free alternative.

Towards being responsible for the environment, reducing the usage of plastic, water and reducing carbon footprint, creation of sustainable, solid hair color protect composition plays a vital role. There is a need to invent such natural, solid hair-care compositions which are sustainable to environment protects hair and applied hair color against damaging UV radiation and provides excellent ease of application to the consumers when mixed with water at the time of use.

## OBJECTIVES OF THE INVENTION

It is an objective of present invention to prepare solid hair color and UV protection composition for making sustainable hair-care formulations that provides excellent ease of application on hair to the consumer when mixed with water at the time of use.

It is an objective of the present invention to prepare mild, natural, silicone free and solid hair color and UV protection compositions for making sustainable hair-care formulations which on heating and molding are used for preparing the final product like bar and powder form.

It is an objective of the present invention to prepare solid hair color protect composition making sustainable hair-care formulations that help in reducing water wastage and excessive usage of plastic.

Another objective of present invention to prepare solid hair color and UV protection composition for making sustainable hair-care formulations to provide superior hair conditioning property.

Yet another objective of invention to prepare solid hair color protect composition for making sustainable hair-care formulations that would smoothen the damaged surface on the hair.

It is another objective of the present invention to prepare solid hair color and UV protection composition for making hair-care formulations for protecting hair and applied color on the hair from harmful UV radiation without imparting any harmful effect on hair and scalp.

## SUMMARY OF INVENTION

The present invention relates to solid hair color and UV protection composition for making sustainable hair-care formulations which when mixed with water at the time of use provide excellent ease of application on hair to the consumers. The solid hair color and UV protection compositions of the present invention are mild, natural, sustainable and provides hair protection against harmful UV radiation thereby imparting hair color and UV protection benefits along with enhanced conditioning.

The solid compositions of the present invention perfectly address the need of the marketplace to provide sustainable hair color and UV protection as well as conditioning benefits. It melts around 80 °C and molded to form waterless, solid conditioning products like hair-color protect bar and powder.

Solid hair color and UV protection compositions of the present invention comprising of water-dispersible, substantive, quaternary ammonium compound, *p*-methoxy cinnamidopropyl behenyl dimethyl ammonium chloride of Formula I where R is a linear alkyl group with minimum of 80 % behenyl (C 22). *p*-methoxy cinnamidopropyl behenyl dimethyl ammonium chloride is synthesized

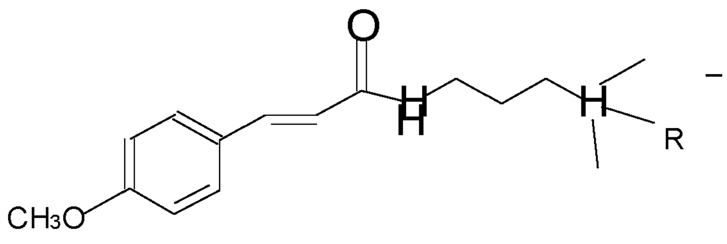

Formula I

wherein p-methoxy cinnamoyl chloride (Formula III) is reacted with *N,N*-dimethyl aminopropylamine under aqueous Schotten Baumann conditions to give the intermediate of *p*-methoxy cinnamidopropyl dimethyl amine (Formula II), the intermediate of Formula II is quaternized with behenyl chloride in aqueous medium

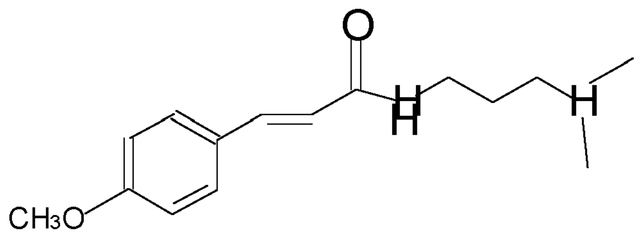

Formula II

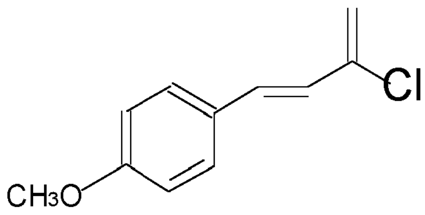

Formula III

*p*-methoxy cinnamidopropyl behenyl dimethyl ammonium chloride is blended with one or more structuring agent like fatty alcohol, natural conditioning agents like butters, natural vegetable/fruit/ nut oil and its corresponding esters, and optionally cationic surfactants, natural antioxidants and preservatives to obtain the solid hair-color protect composition.

In another embodiment, the present invention also relates to a solid hair-color protect composition which can be heated around 80 °C and molded to prepare hair-care bar/powder and such composition are mild, natural and sustainable.

Hence, the solid compositions of the present invention perfectly address the need of the sustainable hair color and UV protection as well as conditioning benefits along with protection of hair from Sun damage.

## DETAILED DESCRIPTION OF INVENTION

It is evident from the background that there are no such sustainable solid hair color and UV protection compositions available in the market so as to meet the sustainability criteria and excellent/superior hair protection/applied hair color and UV protection against UV radiation. Hence, there is a need to develop solid hair-care compositions to provide protection against harmful UV radiation as well as excellent conditioning property.

These solid compositions are prepared by the blend of *p*-methoxy cinnamidopropyl behenyl dimethyl ammonium chloride, one or more structuring agent like fatty alcohol, natural conditioning agents like butters, natural vegetable/fruit/ nut oil and its corresponding esters, cationic surfactants, natural antioxidants and preservatives

These solid compositions also help in reducing water wastage, avoiding the use of excessive plastic hence being

sustainable, ecofriendly and mild, conditioning and protects hair from harmful UV radiation along with better color protection and conditioning effect. The inventors of the present invention have designed solid hair color and UV protection composition comprising water-dispersible, substantive, quaternary ammonium compound, *p*-methoxy cinnamidopropyl behenyl dimethyl ammonium chloride of Formula I where R is a linear alkyl group with minimum of 80 % behenyl (C 22).

*p*-methoxy cinnamidopropyl behenyl dimethyl ammonium chloride is synthesized by

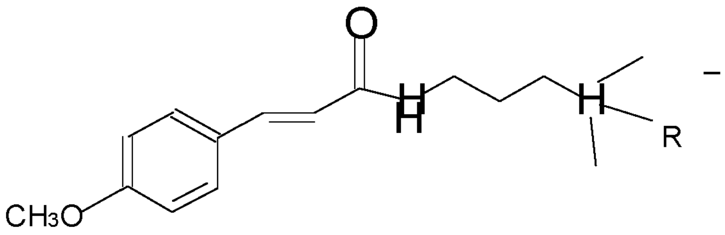

Formula I

reacting p-methoxy cinnamoyl chloride (Formula III) with *N,N*-dimethyl aminopropylamine under aqueous Schotten Baumann conditions to give the intermediate of *p*-methoxy cinnamidopropyl dimethyl amine (Formula II), the intermediate of Formula II is then quaternized with behenyl chloride in aqueous medium.

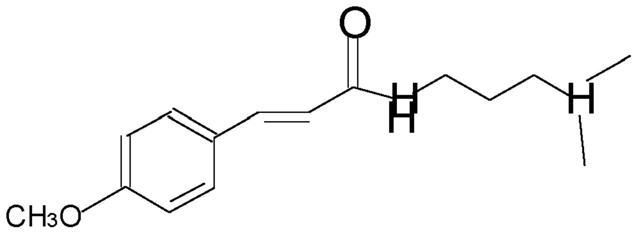

Formula II

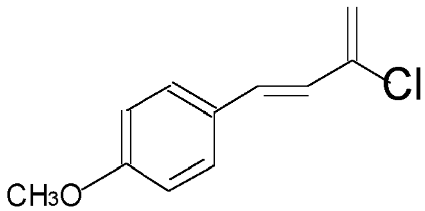

Formula III

and is blended with one or more structuring agent like fatty alcohol, natural conditioning agents like butters, natural vegetable/fruit/ nut oil and its corresponding esters, cationic surfactants, natural antioxidants and preservatives

In another embodiment of the present invention the inventors have unexpectedly found that *p*-methoxy cinnamidopropyl behenyl dimethyl ammonium chloride prepared /synthesized as per the process of Koshti *et. al* when blended with one or more structuring agent like fatty alcohol, natural conditioning agents like butters, natural vegetable/-fruit/ nut oil and its corresponding esters, cationic surfactants, natural antioxidants and preservatives; results in a solid composition which is mild, natural, sustainable which can be heated up to 80 °C and further molded to form hair color and UV protection bars and powder.

Thus, the present invention provides a novel solid hair color and UV protection compositions which are mild, natural and sustainable which when heated around 80 °C and molded forms a homogenous hair-care conditioning UV protector bars and powder.

The present invention provides a mild, natural, sustainable, silicon-free, waterless, solid hair color protection and UV protection composition comprising of water-dispersible, quaternary ammonium and UV active compound, p-methoxy cinnamidopropyl behenyl dimethyl ammonium chloride of Formula I present in an amount between 0.5 to 15 % by weight of total composition,

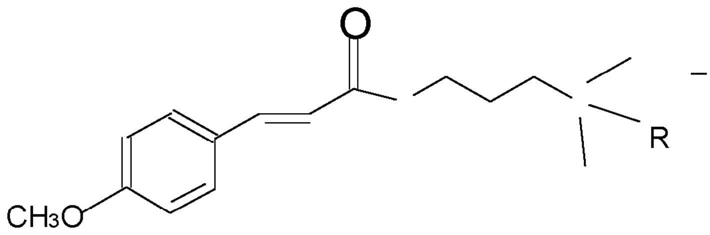

Formula I

where R is a linear alkyl group with minimum of 80 % behenyl (C22),

one or more structuring agents in an amount of 30 to 80% by weight of the total composition,
a natural conditioning agent present in an amount from 1 to 20 % by weight of the total composition,
a natural vegetable/fruit/ nut oil and its corresponding esters present in an amount between 0.5 - 5 % by weight of the total composition,
wherein the pH of the solid hair color protection composition is 3.5 to 5.5;
wherein the solid hair color protection composition provides excellent ease of application when mixed with water at the time of use.

Accordingly, the present invention relates to solid hair color and UV protection compositions comprising: water-dispersible, substantive, quaternary ammonium compound at concentration 0.5 to 15% by weight of total composition which is *p*-methoxy cinnamidopropyl behenyl dimethyl ammonium chloride of Formula I where R is a linear alkyl group with minimum of 80 % behenyl (C 22)

The novel solid compositions of the present invention provide excellent ease of application on hair when mixed water at the time of use, also have high % natural index and high % sustainability quotient.

The present invention also provides a method of preparing the solid hair color protection and UV protection composition, the method comprising the steps of:

a. blending p-methoxy cinnamidopropyl behenyl dimethyl ammonium chloride of Formula I with one or more structuring agents, natural conditioning agent, natural vegetable/fruit/ nut oil and its corresponding esters;
b. heating the blend of step (a) at temperature above 80 °C to form a homogenous waterless hair-care formulation;
c. molding the homogenous waterless hair-care formulation of step (b) to obtain the solid hair color protection and UV protection composition.

**Component A) Color / UV protector agent:**

The solid composition of present invention contains hair color protection as well as UV protection agents. Color protector contribute to the protection of applied hair color on hair while UV protector agents enhance the protection of hair from harmful UV radiations. Based on chemical structures, the Color / UV protector agents can perform other role (e.g. surfactants) also in the present invention. The color and UV protector used for present invention is *p*-methoxy cinnamidopropyl behenyl dimethyl ammonium chloride of Formula I at concentration range from 0.5 to 15% by weight of total weight of the composition. *P*-methoxy cinnamidopropyl behenyl dimethyl ammonium chloride is intentionally used in present invention as none of other color protector and/or UV protector provides the desired combined effects in solid formats.

**Component B) Structuring Agents**

The solid hair color and UV protection composition of present invention contains structuring agents. Structuring agents are the materials which contribute to solid state of composition. Depending upon the type and chemical structure they may perform additional role in composition of present invention. They may lead to desired water solubility so that optimum amount is used for delivering content on hair substrate during application or they may also extend additional hair conditioning benefit.

Fatty alcohols are one of the preferred structuring agents. Fatty alcohol component consists of a fatty alcohol or an alkoxylated fatty alcohol or a mixture of both. Representative fatty alcohols comprise from 8 to 22 carbon atoms, more preferably 16 to 22. Examples of suitable fatty alcohols include cetyl alcohol, stearyl alcohol, cetostearyl alcohol, behenyl alcohol and mixtures thereof. Alkoxylated, (e.g. ethoxylated or propoxylated) fatty alcohols having from about 12 to about 22 carbon atoms in the alkyl chain can be used in place of, or in addition to, the fatty alcohols themselves. Suitable

examples include ethylene glycol cetyl ether, polyoxyethylene (2) stearyl ether (steareth 2), polyoxyethylene (4) cetyl ether (ceteth 4), and mixtures thereof. The preferred structuring agent includes cetostearyl alcohol at concentration 30 to 80% by weight of total composition and steareth 2 at concentration of 1 to 40% of total bar composition used alone or in combination.

**Component C) Oily Conditioning agent**

The inventive composition contains an oil conditioning component at concentration 1% to 20% by weight. These contribute for enhanced conditioning, moisturization & nourishment of hair substrate. These can be liquid, pasty, semisolid or solid form used individually as single component or in combination.

These include different fatty esters which are mono-, di- and triglycerides. Some of the key triglycerides which are part of present composition are butters like shea butter, cocoa butter, murumuru butter, mango seed butter, olive butter, hemp seed butter, almon butter, coconut butter, kokum butter, babasu butter, macadamia butter or material of similar nature obtained from vegetable origin. Synthetic butters equivalent to natural ones produced by modification and processing of vegetable oils can also be used in present invention. The preferred butters which are the part of present invention are shea butter & cocoa butter at concentration of 1 to 20% by weight.

Oily conditioning components further include variety of vegetable oils, such as coconut oil, castor oil, safflower oil, sunflower oil, cottonseed oil, corn oil, olive oil, soyabean oil, almond oil, avocado oil, palm oil or its fractions palm olein or palm stearin, sesame oil, peanut oil and lanolin. Partially or fully hydrogenated derivatives of these components can also be part of composition of present invention. Castor oil, coconut oil, almond oil, olive oil are the ones which are preferably used at concentration of 0.5 to 5% by weight.

Esters of long chain fatty acids like glyceryl monostearate, glyceryl monolaurate, ethylene glycol monostearate, ethylene glycol distearate, polyglyceryl esters of fatty acid like polyglycerol 2 stearate, polyglycerol 4 laurate, polyglycerol 3 oleate, polyethylene glycol esters of fatty acids like polyethylene glycol 150 distearate, cocomonoethanolamide can be used as oily hair conditioning component in present invention. These are optional component in the present invention and can be in range of 1 to 20 % by weight of the final composition.

**Component D) Conditioning Surfactant (optional):**

The solid hair conditioner compositions of the present invention comprise one or more hair conditioning surfactants. Suitable hair conditioning surfactants are selected from cationic surfactants, used singly or in admixture. These includes alkyltrimethylammonium chlorides wherein the alkyl group has from about 8 to 22 carbon atoms, for example octyltrimethylammonium chloride, dodecyltrimethylammonium chloride, hexadecylt rimethylammonium chloride, cetyltrimethylammonium chloride, octyldimethylbenzylammonium chloride, decyldimethylbenzylammonium chloride, Stearyldimethyl benzylammonium chloride, didodecyldimethylammonium chloride, dioctadecyldimethylammonium chloride, tallow trimethylammonium chloride, cocotrimethylammonium chloride, di-hardened tallow dimethylammonium chloride, distearyldimethylammonium chloride, PEG-2 oleamonium chloride, behenyltrimonium chloride and the corresponding salts thereof,

e.g., bromides, hydroxides, Cetylpyridinium chloride at concentration range of 0.1% to 20% by weight. Behentrimonium methosulfate, behenamidopropyltrimonium methosulfate. The behentrimonium-type cationic surfactants may be used singly or in combination of two or more.

The conditioning surfactants can also further be selected from stearamidopropyldimethylamine, behnamidopropyldimethylamine, cocoamidopropyldimethylamine at concentration of 0.1 to 20% by weight in present invention.

**Component E) Optional Ingredients**

Compositions of this invention may contain additional ingredients generally used in hair conditioners. These other ingredients may include additional conditioning agents, preservatives, polyols such as glycerine and polypropylene glycol, chelating agents, pH adjusting components, colouring agents and fragrances.

**Cationic Polymers**

The compositions according to the present invention may comprise a cationic polymer for further enhancing hair conditioning efficacy of bars prepared from present invention. Some of the examples which can be part of present composition are copolymers of 1-vinyl-2-pyrrolidine and 1-vinyl-3-methyl-imidazolium Salt (e.g. chloride Salt), referred to in the industry by the Cosmetic, Toiletry, and Fragrance Association, (CTFA) as Polyguaternium-16; copolymers of 1-vinyl-2-pyrrolidine and dimethylaminoethyl methacrylate, referred to in the industry (CTFA)as Polygulaternium-11; cationic diallyl quaternary ammonium-containing polymers including, for example, dimethyldially ammonium chloride

homopolymer and copolymers of acrylamide and dimethyldiallylammonium chloride, referred to in the industry (CTFA) as Polyquaternium 6 and Polyquaternium 7, respectively.

Other cationic conditioning polymers that can be used include cationic polysaccharide polymers, Such as cationic cellulose derivatives, cationic Starch derivatives, and cationic guar gum derivatives.

Cationic cellulose which are salts of hydroxyethylcellulose reacted with trimethyl ammonium substituted epoxide, referred to in the industry (CTFA) as Polyguaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyguaternium 24.

A particularly Suitable type of cationic polysaccharide polymer that can be used is a cationic guar gum derivative, such as guar hydroxypropyltrimonium chloride.

Preservatives suitable for the present invention is selected from carboxylic acid (in free acid form) or their salts chosen from salts of benzoic acid, propionic acid, salicylic acid, sorbic acid, 4-hydroxybenzoic acid, dehydroacetic acid, formic acid, p-methoxybenzoic acid, acetic acid, or 10-undecylenic acid, lactic acid, 25 undecenoic acid, glycolic acid, maleic acid, malic acid, citric acid, gluconic acid and their salts. These are optional component in the present invention and can be in range of 0.1 to 3 % by weight of the final composition.

The pH of present invention is adjusted using citric acid, lactic acid, glycolic acid or any of suitable acidifying agent. The concentration of acidifying agents ranges between 0.01 to 3% by weight.

The hair color protect composition of present invention may contain chelating agents like, Disodium Etidronate, Sodium Gluconate, Tetrasodium glutamate diacetate, Ethylenediaminetetraacetic Acid or its Sodium salt.

Skin care active & anti-oxidant like Vitamin E Acetate can used as an optional ingredient in present invention at concentration range between 0.01 to 1% by weight.

The performance characteristics of the present novel solid hair color protect compositions are explained as below.

**Hair Color protection:**

The novel solid hair color and UV protection composition protects applied hair color on hair and provides good hair color protection property. Hair color and UV protection bar composition of Example 1 of the present invention, is the only composition showing significant hair color protection when compared with the composition of Example 4, Example 5 and Example 7.

**Hair UV protection:**

The novel solid hair color and UV protection composition protects hair from harmful UV radiation. Hair color and UV protection bar composition of Example 2 of the present invention, is the only composition showing significant hair UV protection as when compared with the composition Example 4, Example 5, Example 7.

**Combing Force Reduction:**

The novel solid hair color and UV protection composition is substantive and advantageously provides good conditioning there by combing force reduction on hair.

Hair color and UV protection bar composition of Example 3 of the present invention shows a significant improvement in the wet and dry combing force reduction property as compared to the composition Example 5, Example 6 and Example 7.

**Sensory Benefits:**

Sensory Evaluation is defined as *"A scientific discipline used to evoke, measure, analyze, and interpret those responses to products that are perceived by the senses of sight, smell, touch, taste, and hearing"*

Solid hair color and UV protection composition of the Example 3 of the present invention shows a significant improvement in the sensorial property as compared to market brand of Example 7.

**Wear Rate Study:**

The usage study of solid hair color and UV protection composition of Example 2 of the present invention was done against liquid rinse off conditioner of Example 6 with an objective to compare solid versus liquid formats of hair conditioners. As shown in Figure 6, the solid hair color and UV protection composition has very less consumption in single wash cycle as compared to liquid counterpart.

**Advantages of the invention:**

1. The solid hair color and UV protection compositions of the present invention can be heated around 80-90°C to form homogenous waterless hair-care formulations. Hence, is very much compatible in the formulations requiring heat treatment.
2. The solid hair color and UV protection compositions of the present invention provides excellent ease of application to the consumer when mixed with water at the time of use.
3. The solid hair color and UV protection compositions of the present invention are mild and also reduce wastage of water and excessive usage of plastic. Hence are sustainable.
4. The solid hair color and UV protection compositions of the present invention have high naturality index which provides highly natural hair care compositions having more than 80% naturalness as per ISO section 5.2.2 of ISO 16128-2.
5. The solid hair color and UV protection compositions of the present invention have superlative conditioning property and suitable for making sustainable hair-care formulations
6. The solid hair color and UV protection compositions would smoothen the damaged surface providing excellent hair sensory.
7. The solid hair color and UV protection compositions protect hair and applied color on the hair from harmful UV radiation without imparting any negative effect on the hair and scalp.

**Examples:**

The present invention is now described by way of working non-limiting illustrative examples. These examples are provided for illustrative purposes only and are not intended to limit the scope of the invention.

The raw materials used in preparation of hair color protection, UV protection composition bars of the present invention are mentioned as below.

| Raw Materials | Supplier |
|---|---|
| Cetostearyl alcohol | Godrej Industries Ltd with trade name Ginol16-18, 50:50 |
| Steareth 2 | Godrej Industries Ltd with trade name Ginonic S2 |
| Shea Butter | Rolex Lanolin India, with trade name RolanSHEAB-R |
| Cocoa Butter | TRI-K Industries, USA with trade name Tomasa Cocoa Butter (NF) |
| Behentrimmonium Chloride | Thor with trade name Microcare Quat BHQ |
| Stearamidopropyl Dimethylamine | Thor with trade name Microcare Amide STAM |
| Castor Oil | Ambuja Solvex Pvt.Ltd. Trade Name: Castor Oil Deodorized-ULTRA |
| Vitamin E Acetate | Matrix Life Science Private Limited, Trade Name: d-Alpha Tocopheryl Acetate 1 000IU Non-GMO Soy (IP) |
| Lactic Acid | Corbion Make supplied With Trade Name Purac HS 88 |
| Glyceryl Monostearate | Fine Organics, Trade Name: GMS S/W Flakes |
| Hydroxyethylcellulose | Ashland, Trade Name: Natrosol 250 HHR-P |
| Keratin NPNF | TRI-K Industries USA |

**Example 1: Solid hair color protection conditioning bar with methoxycinnamidopropyl behendimmonium chloride for hair color protection efficacy demonstration.**

**Table 1**

| INCI Name/Chemical Name | % (W/W) |
|---|---|
| Cetostearyl Alcohol | 45.08 |
| Steareth 2 | 32.87 |
| Shea Butter | 9.75 |
| Methoxycinnamidopropyl Behendimonium Chloride (MCBDC) | 10.00 |
| Castor Oil | 2.13 |
| Lactic Acid | 0.07 |
| Vitamin E Acetate | 0.10 |

Procedure: In a glass beaker, required quantities of cetostearyl alcohol, steareth 2, sheabutter were charged. The content in a beaker was melted by heating to 80-90°C under continuous stirring. MCBDC needles, castor oil, lactic acid & vitamin E acetate were subsequently added. The molten mass in a beaker was mixed continuously at 80-90°C until MCBDC needles were melted and uniformly mixed. It was ensured that all raw materials were uniformly mixed and no undissolved particles remains in molten mass of beaker. After uniformity was achieved the molten mass at 80-90°C was poured on clean stainless steel (SS 316) tray and allowed to cool and solidify to generate flakes of inventive composition.
The flakes of inventive composition of the present invention were melted in separate glass beaker under continuous stirring at 80-90°C. Fragrance can be added as per requirement and mixed. The resultant molten mass was poured in molds to obtain hair color and UV protection conditioning bar composition. The pH of 5% aqueous dispersion by weight was 4.41.

**Example 2: Solid hair color protection conditioning bar with methoxycinnamidopropyl behendimmonium chloride for hair tensile strength studies.**

**Table 2**

| INCI Name/Chemical Name | % (W/W) |
|---|---|
| Cetostearyl Alcohol | 66.20 |
| Cocoa Butter | 10.00 |
| Steareth 2 | 6.50 |
| Glyceryl Monostearate | 10.00 |
| Methoxycinnamidopropyl Behendimonium Chloride (MCBDC) | 0.80 |
| Behentrimmonium Chloride | 1.20 |
| Stearamidopropyl Dimethylamine | 1.00 |
| Castor Oil | 4.10 |
| Lactic Acid | 0.10 |
| Vitamin E Acetate | 0.10 |

Procedure: In a glass beaker, required quantities of cetostearyl alcohol, cocoa butter, steareth 2, glyceryl monostearate were charged. The content was melted by heating to 80-90°C under continuous stirring. MCBDC needles, behentrimmonium chloride, stearamidopropyl dimethylamine, castor oil, lactic acid & vitamin E acetate were subsequently added. The molten mass in a beaker was mixed continuously at 80-90°C until MCBDC needles were melted and uniformly mixed. It was ensured that all raw materials were uniformly mixed and no undissolved particles remains in molten mass of beaker. After uniformity was achieved the molten mass at 80-90°C was poured on clean stainless steel (SS 316) tray and allowed to cool and solidify to generate flakes of inventive composition.
The flakes of composition were melted in separate beaker under continuous stirring at 80-90°C. Fragrance can be added as per requirement and mixed. The resultant molten mass was poured in molds to obtain hair color and UV protection conditioning bar.

The pH of 5% by weight of aqueous dispersion of the bar was 4.9.

**Example 3: Solid hair color protection conditioning bar with methoxycinnamidopropyl behendimmonium chloride for conditioning efficacy study**

**Table 3**

| INCI Name/Chemical Name | % (W/W) |
|---|---|
| Cetostearyl Alcohol | 60.08 |
| Steareth 2 | 22.87 |
| Shea Butter | 9.75 |
| Methoxycinnamidopropyl Behendimonium Chloride (MCBDC) | 5.00 |
| Castor Oil | 2.13 |
| Lactic Acid | 0.07 |
| Vitamin E Acetate | 0.10 |

Procedure: In a glass beaker, required quantities of cetostearyl alcohol, steareth 2, shea butter were charged. The content was melted by heating to 80-90°C under continuous stirring. MCBDC needles, castor oil, lactic acid & vitamin E acetate were subsequently added. The molten mass in a container was mixed continuously at 80-90°C until MCBDC needles were melted and uniformly mixed. It was ensured all raw materials were uniformly mixed and no undissolved particles remains in molten mass of container. After uniformity was achieved the molten mass at 80-90°C was poured on clean stainless steel (SS 316) tray and allowed to cool and solidify to generate flakes.

The flakes of the composition were melted in separate container under continuous stirring at 80-90°C. Fragrance can be added as per requirement and mixed. The resultant molten mass was poured in molds to obtain hair color and UV protection conditioning bar of inventive composition.

The pH of 5% by weight of aqueous dispersion of the bar was 4.12.

**Example 4: Solid hair color protection conditioning bar with keratin active as a hair color protecting agent demonstrated in commercially available market color protection keratin conditioning bar as "Tonirose" for hair color protection & relevant performance study**

**Table 4**

| INCI Name/Chemical Name | % (W/W) |
|---|---|
| Cetostearyl Alcohol | 45.12 |
| Steareth 2 | 32.87 |
| Shea Butter | 9.75 |
| Keratin NPNF | 10.00 |
| Castor Oil | 2.13 |
| Lactic Acid | 0.03 |
| Vitamin E Acetate | 0.10 |

Procedure: In a glass beaker, required quantities of cetostearyl alcohol, steareth 2, shea butter were charged. The content was melted by heating to 80-90°C under continuous stirring. Keratin NPNF, castor oil, lactic acid & vitamin E acetate were subsequently added. The molten mass in a beaker was mixed continuously at 80-90°C. It was ensured all raw materials were uniformly mixed and no undissolved particles remains in molten mass of beaker. After uniformity was achieved the molten mass at 80-90°C was poured on clean stainless steel (SS 316) tray and allowed to cool and solidify to generate flakes for preparation of keratin based hair color protection bar.

The flakes of keratin based hair color protection bar were melted in separate beaker under continuous stirring at 80-90°C. Fragrance was added as per requirement and mixed. The resultant molten mass was poured in molds to obtain keratin based hair color protection conditioning bar. The pH of 5% by weight of aqueous dispersion of bar was 4.35.

**Example 5: Placebo hair conditioning bar without methoxycinnamidopropyl behendimmonium chloride as comparative example against solid hair color protection bar of the present invention**

**Table 5**

| INCI Name/Chemical Name | % (W/W) |
|---|---|
| Cetostearyl Alcohol | 62.58 |
| Steareth 2 | 25.37 |
| Sheabutter | 9.75 |
| Castor Oil | 2.13 |
| Lactic Acid | 0.07 |
| Vitamin E Acetate | 0.1 |

Procedure: In a beaker, required quantities of cetostearyl alcohol, steareth 2, shea butter were charged. The content was melted by heating to 80-90°C under continuous stirring. Castor oil, lactic acid & vitamin E acetate were subsequently added. The molten mass in a beaker was mixed continuously at 80-90°C and uniformly mixed. It was ensured all raw materials were uniformly mixed and no undissolved particles remains in molten mass of container. After uniformity was achieved the molten mass at 80-90°C was poured on clean stainless steel (SS 316) tray and allowed to cool and solidify to generate flakes of placebo composition.
The pH of 5% by weight of aqueous dispersion of bar was. 3.80
The flakes of placebo composition were melted in separate container under continuous stirring at 80-90°C. Fragrance can be added as per requirement and mixed. The resultant molten mass was poured in molds to obtain placebo hair conditioning bar without Methoxycinnamidopropyl Behendimmonium Chloride

**Example 6: Rinse off liquid hair conditioner containing methoxycinnamidopropyl behendimmonium chloride mentioned in US 9,463,337 B2.**

**Table 6**

| INCI Name/Chemical Name | % (W/W) |
|---|---|
| Water | To make 100.00 |
| Hydroxyethylcellulose | 0.50 |
| Cetostearyl alcohol | 3.00 |
| Methoxycinnamidopropyl Behendimonium Chloride (MCBDC) | 2.00 |
| Phenoxyethanol | 0.50 |

Procedure: In a glass beaker weigh required quantity of water and charge hydroxy ethyl cellulose under stirring. Heat this phase to 80-85°C. In a separate beaker weigh cetostearyl alcohol, MCBDC, Phenoxyethanol & heat it to 80-85°C till its melted. Pour this hot molten mass in aqueous phase maintained at 80-85°C and stir it for 10 minutes. Gradually cool down the temperature of the mixture to obtain Rinse off liquid hair conditioner containing MCBDC.
The pH of liquid rinse off hair conditioner was 4.44.

**Example 7: Leading Hair Conditioning Bar Marketed with Brand Name Ethique.**

The readily available market brand was used as a comparative example against inventive composition of the

present invention. The pH of 5% by weight of aqueous dispersion was 4.61.

**Hair Color protection of compositions of present invention:**

Fig. 1 illustrates the ΔE (hair color loss) of the hair tresses treated with solid hair color protect composition of Example 1 and solid hair color protection composition of Example 4, Example 5 and Example 7 respectively. The average hair color loss i.e. ΔE of the solid hair color protection composition of present invention Example 1 is substantially lower than composition of Example 4, 5 and 7. Hence it is proved that the novel solid hair color protection composition of present invention has an excellent hair color protection property of applied color on the hair.

**Hair Color Protection Measurement Procedure:**

Double bleached European hair were pre-treated (150 cm long, 5 g) with 10 % SLES and dried. They were dyed using **L'Oreal's Excellence Hicolor Red H11 Intense Red** as per the protocol described by the dye manufacturer.

Hair coloring (hair dyeing) process is performed as recommended by the marketed product. The color and the developer were mixed in the recommended ratio the mixture was applied uniformly to the hair tresses and covered with the aluminum foil for thirty minutes. After this the hair tresses were washed with warm water. The colored hair tresses were divided into various groups for test samples treatment for hair color protection with Example 1, Example 4, Example 5 and Example 7 respectively.

All respective hair tresses were exposed to UV radiation, total exposure of 2.5 hours through 5 washing cycles. Each washing cycle included application of test sample for 90 strokes on each side of pre-wet hair tress. After test sample was applied, hair tress was gently rubbed with fingers for uniform distribution and coverage on hairs for 3 minutes. Further hair tress was given 5 minutes contact time and subsequently rinsed with water for 1 minute. Hair tresses were then exposed to UV light for 30 minutes.

The treated tresses were irradiated with UV chamber which is equipped with two tubes emitting UV light in short UV (254 nm) and long UV 315-400 nm) regions. Sankyo Denki G8TS tube with UV output of 2.5 W was used as short UV light source while Sankyo Denki F8T5BLB backlight -blue tube with UV output of 1.4 W was used as long UV light source.

The fading of color of the sets of hair tresses labelled as Example 1, Example 4, Example 5 & Example 7 was measured on the Hunter instrument, Labscan XE spectrophotometer. The total color loss (ΔE) is assessed by the change in L* a* b* scale using the equation $\Delta E = [(\Delta L)^2 + (\Delta a)^2 + (\Delta b)^2]^{1/2}$.

The results are summarized in Fig 1.

The ΔE was lower in case of the inventive composition Example 1 as compared with Example 4 with Keratin as color protecting agent, Example 5 a placebo composition & Example 7 market hair conditioning bar signified anti fade activity of Example 1. The comparative examples, Example 4, Example 5 & Example 7 did not show anti-fade activity at all.

**UV protection of hair using compositions of present invention:**

Fig. 2 illustrates the total force (gmf) required to break hair fiber of the hair tresses treated with solid hair color protection composition of Example 2 of the present invention with comparative Example 4, Example 5 and Example 7. The average force required to break hair fiber of the solid hair color protection composition of Example 2 of the present invention is substantially higher than comparative Example 4, Example 5, Example 7. Hence it proves that the solid hair color protection composition of present invention does not weaken the hair surface on UV exposure instead protects hair from UV damage.

**Measurement Procedure of protection of hair from UV damage:**

Double bleached European hair were pre-treated (150 cm long, 1.5 g) with 10 % SLES and dried. Pretreated hair tresses were treated with hair color protection bar inventive composition of Example 2, comparative composition of Example 4, Example 5 and Example 7 respectively.

All respective hair tresses were exposed to UV radiation, total exposure of 2.5 hours through 5 washing cycles. Each washing cycle included application of test sample for 90 strokes on each side of pre wet hair tress followed by gently rubbing with fingers for uniform distribution and coverage on hairs for 3 minutes. Further hair tress was given 5 minutes contact time followed by rinsing with water for 1 minute. Hair tress were than exposed to UV light for 30 minutes. After washing cycle & UV exposure, 50 hair fibres of respective test samples were crimped and subjected to tensile testing on Diastron Tensile Tester.

Each crimped fiber is pulled apart and the force required to break each fiber is measured by instrument software.

Average force measured as Mean Breakload (gmf) required to break hair fibers is calculated and plotted. Higher the force required lesser is the UV damage and more is the UV protection. Mean Breakload (gmf) in case of the solid hair

color protect composition of present invention Example 2 is substantially higher than solid hair color protects composition of Example 4, placebo composition Example 5 and market commercially available hair conditioning bar Example 7. This showed significant UV protection activity of hair color protect bar of inventive Example 2 as shown in figure 2 below.

**Reduction in Combing force using composition of present invention:**

Fig. 3 illustrates the combing force reduction of the hair tresses treated with solid hair color protection composition of Example 3 of the present invention with comparative Example 5, Example 6 and Example 7. The wet and dry combing force reduction of the solid hair color protection composition of Example 3 of the present invention. is substantially higher than comparative Example 5, Example 6 and Example 7.

**Conditioning/Combing Force Reduction Procedure:**

Double bleached European hair were pre-treated (150 cm long, 1.5 g) with 10 % SLES and dried. The combing resistance was then measured in terms of the force required to pull a comb through a tress of a hair. Each tress was combed on the Diastron tensile tester (MTT 175). Experimental conditions maintained were a) temperature of 22°C and b) relative humidity of 60 %.

Hard rubber comb was used for the analysis. Ten combing strokes were conducted for each tress and averaged to obtain a representative value. After the average combing force was determined for each wet tress, the tresses were allowed to dry at room temperature for 18 h before average combing force was measured on the dry tresses. These were the initial measurements before treatment of hair tresses with test samples. In the similar manner both wet and dry combing force was measured for the hair tress after treatment with the composition of inventive Example 3, Example 5, Example 6 & Example 7. For treatment of single pre-wet hair tress, test sample was applied for 60 strokes on each side. Further contact time was given for 5 minutes followed by rinsing off with water for 1 minute. 5 hair tresses were considered for each test composition i.e. example 3, example 5, example 6 & example 7 respectively. The test results are summarized as % combing force reduction for the test composition against the untreated tresses. Calculated from the below formula.

$$\% \text{ Reduction in Combing Force} = 100 - \frac{\text{Treated Hair (Total Work Done, Joules)}}{\text{Untreated (total Work Done, Joules)}} \text{X100}$$

As evident in the below Figure 3. the hair tresses treated with the composition of Example 3 exhibits significantly better wet and dry combing force reduction than the comparative placebo composition of Example 5, rinse off conditioner of US 9,463,337 B2 Example 6 and market hair conditioning bar Example 7.

**Sensory Test**

The solid hair color protection bar composition of the present invention provides a very good sensory feel to the hair. The hair tresses were treated as mentioned in above hair combing force reduction study. Inventive Example 3 & comparative Example 6 were compared for hair sensory. Figure -5 illustrates the results of the sensory test and proves that the inventive solid hair color protect composition Example 3 of present invention is significantly better than hair conditioning composition of Example 6.

**Procedure for sensory evaluation**

i. Hair tress treatment procedure is same as the one used in hair combing force reduction study.
ii. Following is the rating scale used and evaluation is done with the help of 20 panel members

Scale 1 is the lowest and scale 5 represents the highest performance.

**Wear Rate Study:**

The usage study of solid hair color protection composition of Example 2 was done against liquid rinse off conditioner of Example 6 with an objective to compare solid versus liquid formats of hair conditioners. 200 grams of hair tresses was pre-treated with 10 % SLES solution. 30 strokes of solid hair colour protection bar of Example 2 and liquid conditioner of Example 6 were applied respectively to the pre-wet hair tresses and were rinsed off. Similarly, 10 cycles of

application were conducted for respective examples and average amount consumed for Example 2 & Example 7 was measured by difference of initial & final weights after every application. As shown in the figure 6, the amount of solid hair protection bar of Example 2 was consumed is significantly less compared to liquid counterpart of US 9,463,337 B2 of Example 6. This study demonstrates solid formats are more sustainable and shall help to reduce water consumption, also helps in reducing discharge of chemicals to the environment & further reduce plastic waste generation.

**Claims**

1. A mild, natural, sustainable, silicon-free, waterless, solid hair color protection and UV protection composition comprising:

   water-dispersible, quaternary ammonium and UV active compound, *p*-methoxy cinnamidopropyl behenyl dimethyl ammonium chloride of Formula I present in an amount between 0.5 to 15 % by weight of total composition,

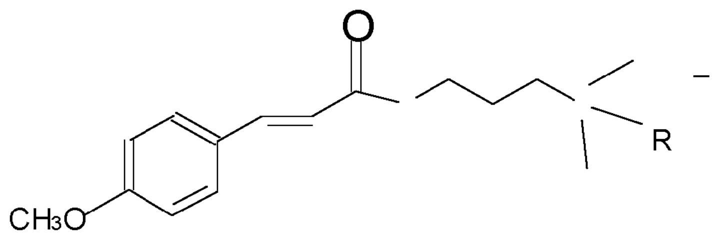

Formula I

   where R is a linear alkyl group with minimum of 80 % behenyl (C22),
   one or more structuring agents in an amount of 30 to 80% by weight of the total composition,
   a natural conditioning agent present in an amount from 1 to 20 % by weight of the total composition,
   a natural vegetable/fruit/ nut oil and its corresponding esters present in an amount between 0.5 - 5 % by weight of the total composition, and
   wherein the pH of the solid hair color protection composition is 3.5 to 5.5.

2. The solid hair color protection and UV protection composition as claimed in claim 1, wherein *p*-methoxy cinnamidopropyl behenyl dimethyl ammonium chloride of Formula I is synthesized by reacting *p*-methoxy cinnamoyl chloride of Formula III with *N,N*-dimethyl aminopropylamine under aqueous Schotten Baumann conditions to give the intermediate of *p*-methoxy cinnamidopropyl dimethyl amine of Formula II and the intermediate of Formula II is quaternized with behenyl chloride in aqueous medium.

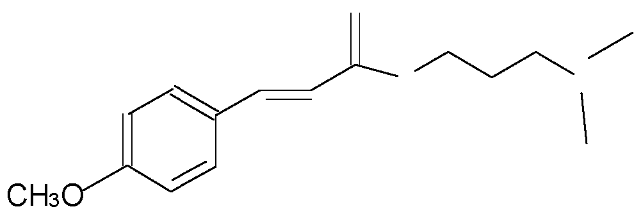

Formula II

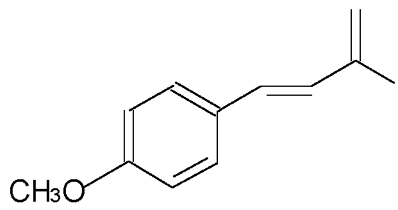

Formula III

3. The solid hair color protection and UV protection composition as claimed in claim 1 or 2, wherein one or more structuring agents are selected from fatty alcohols and/or fatty alcohol ethoxylates.

4. The solid hair color protection and UV protection composition as claimed in claim 3, wherein fatty alcohols are present in the range of 30 - 80 % by weight of the total composition and are selected from cetyl alcohol, stearyl alcohol, cetostearyl alcohol and beheneyl alcohol;
   wherein fatty alcohol ethoxylates are present in the range of 1 - 40 % by weight of the total composition and are selected from steareth 2 and ceteth 4.

**5.** The solid hair color protection and UV protection composition as claimed in any one of the preceding claims, wherein the natural conditioning agent is selected from one or more of shea butter, cocoa butter and mango seed butter.

**6.** The solid hair color protection and UV protection composition as claimed in any one of the preceding claims, wherein the natural vegetable/fruit/ nut oil and its corresponding ester is selected from one or more of castor oil, coconut oil, almond oil and olive oil.

**7.** The solid hair color protection and UV protection composition as claimed in any one of the preceding claims, wherein the composition comprises one or more of cationic surfactants, natural antioxidants, preservatives and fragrances.

**8.** The solid hair color protection and UV protection composition as claimed in any one of the preceding claims, wherein the composition is a hair color, UV protection bar or UV protection powder.

**9.** The solid hair color protection and UV protection composition as claimed in any one of the preceding claims, wherein the solid hair color protection composition provides excellent ease of application when mixed with water at the time of use.

**10.** A method of preparing the solid hair color protection and UV protection composition of any one of the preceding claims, the method comprising the steps of:

a. blending p-methoxy cinnamidopropyl behenyl dimethyl ammonium chloride of Formula I with one or more structuring agents, natural conditioning agent, natural vegetable/fruit/ nut oil and its corresponding esters;
b. heating the blend of step (a) at temperature above 80 °C to form a homogenous waterless hair-care formulation;
c. molding the homogenous waterless hair-care formulation of step (b) to obtain the solid hair color protection and UV protection composition.

Fig. 1

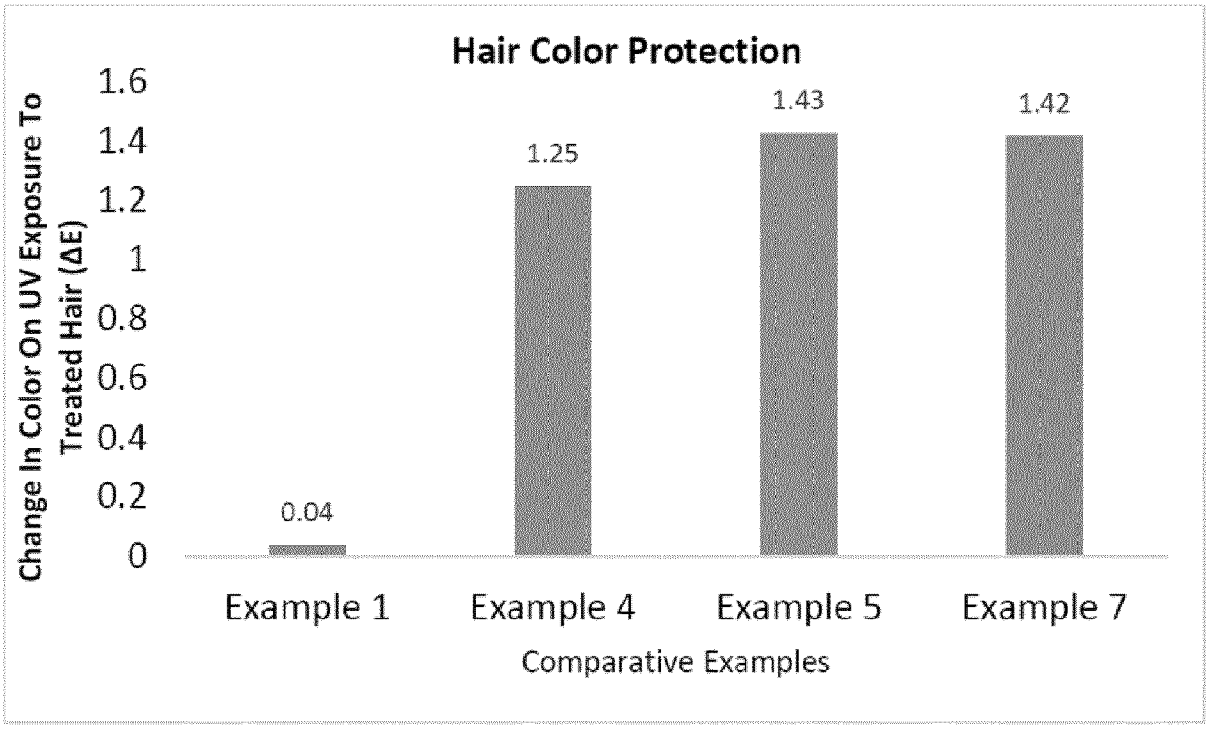
Hair Color Protection
Change In Color On UV Exposure To Treated Hair (ΔE)
1.6
1.4
1.2
1
0.8
0.6
0.4
0.2
0
0.04
1.25
1.43
1.42
Example 1
Example 4
Example 5
Example 7
Comparative Examples

Fig. 2

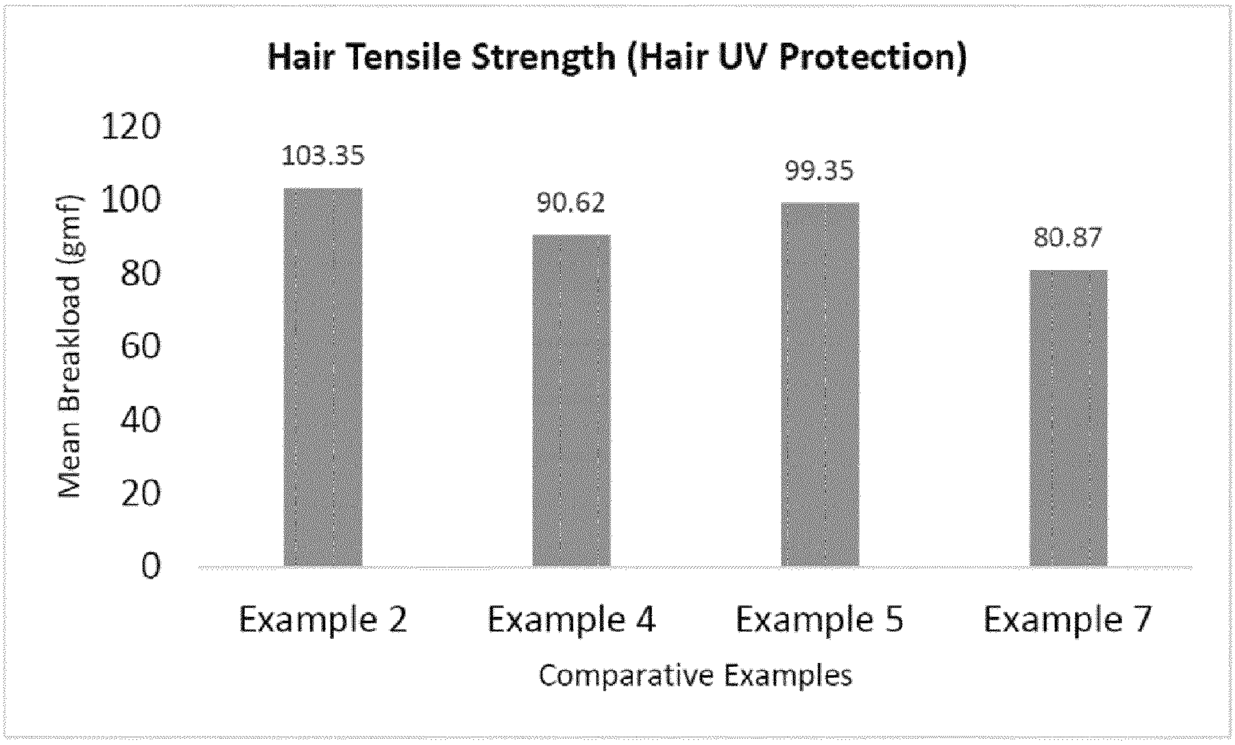
Hair Tensile Strength (Hair UV Protection)
Mean Breakload (gmf)
120
100
80
60
40
20
0
103.35
90.62
99.35
80.87
Example 2
Example 4
Example 5
Example 7
Comparative Examples

Fig. 3

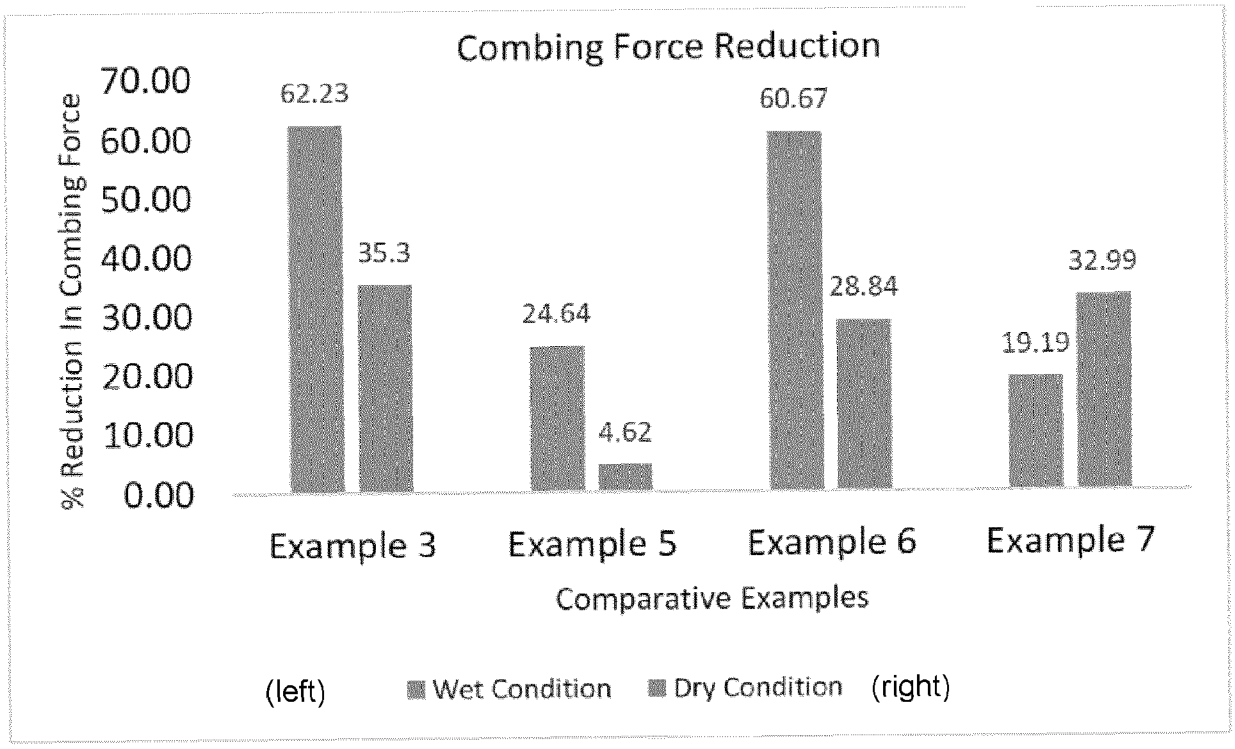
Combing Force Reduction
% Reduction In Combing Force
70.00
60.00
50.00
40.00
30.00
20.00
10.00
0.00
62.23
35.3
24.64
4.62
60.67
28.84
19.19
32.99
Example 3
Example 5
Example 6
Example 7
Comparative Examples
(left)
Wet Condition
Dry Condition
(right)

Fig. 4

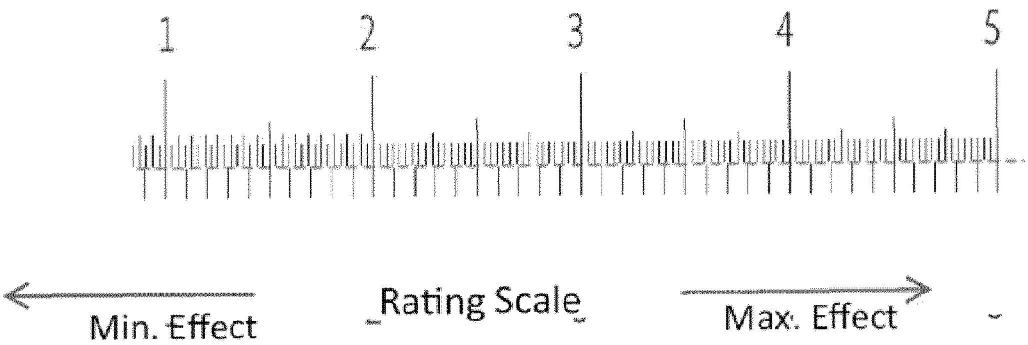
1
2
3
4
5
Min. Effect
Rating Scale
Max. Effect

Fig. 5

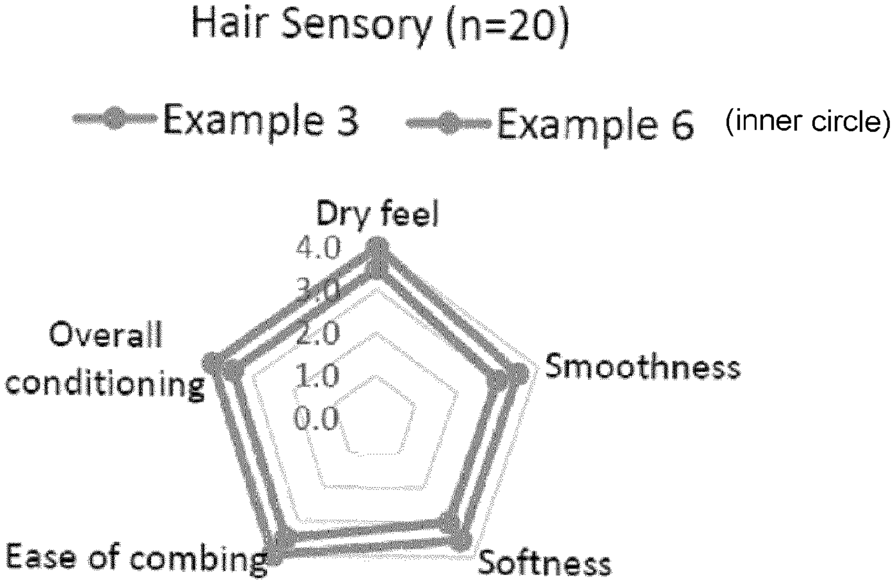
Hair Sensory (n=20)
Example 3
Example 6
(inner circle)
Dry feel
4.0
3.0
2.0
1.0
0.0
Smoothness
Softness
Ease of combing
Overall conditioning

Fig. 6

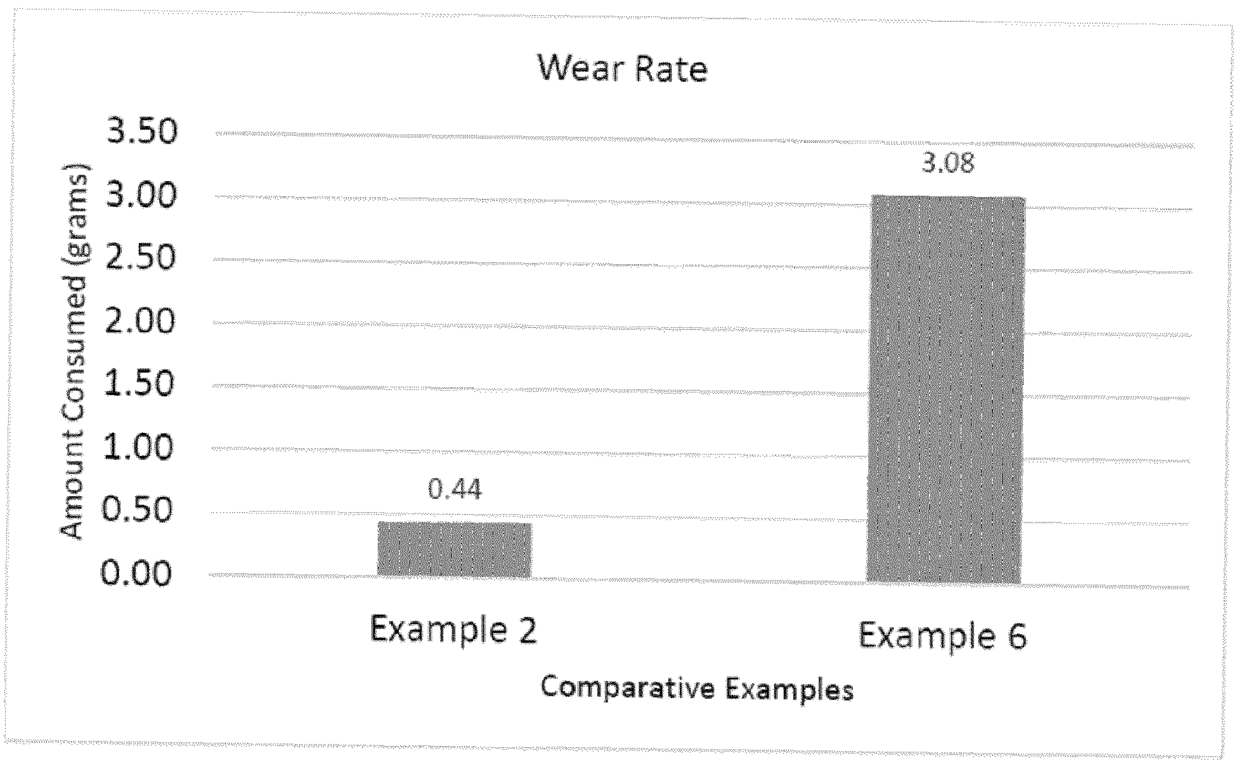
Wear Rate
Amount Consumed (grams)
3.50
3.00
2.50
2.00
1.50
1.00
0.50
0.00
0.44
3.08
Example 2
Example 6
Comparative Examples

Europäisches Patentamt
European Patent Office
Office européen des brevets

# EUROPEAN SEARCH REPORT

Application Number
EP 25 15 0310

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2014/111856 A1 (GALAXY SURFACTANTS LTD [IN]) 24 July 2014 (2014-07-24)<br>* the whole document *<br>----- | 1-10 | INV.<br>A61K8/42<br>A61Q5/00<br>A61Q5/12 |
| A | US 2019/343744 A1 (KOSHTI NIRMAL [US] ET AL) 14 November 2019 (2019-11-14)<br>* paragraph [0013] *<br>----- | 1-10 | |
| A | US 2005/080135 A1 (KOSHTI NIRMAL MADHUKAR [IN] ET AL) 14 April 2005 (2005-04-14)<br>* the whole document *<br>----- | 1-10 | |
| A | Zuzana: "The Ultimate Guide to Solid Conditioner Bars",<br>,<br>10 March 2023 (2023-03-10), XP093269469,<br>Retrieved from the Internet:<br>URL:https://threehillssoap.ie/the-complete-guide-to-solid-conditioner-bars/<br>----- | 1-10 | |

| TECHNICAL FIELDS SEARCHED (IPC) |
|---|
| A61K<br>A61Q |

The present search report has been drawn up for all claims

2

| Place of search | Date of completion of the search | Examiner |
|---|---|---|
| Munich | 5 May 2025 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

# ANNEX TO THE EUROPEAN SEARCH REPORT ON EUROPEAN PATENT APPLICATION NO. EP 25 15 0310

This annex lists the patent family members relating to the patent documents cited in the above-mentioned European search report. The members are as contained in the European Patent Office EDP file on
The European Patent Office is in no way liable for these particulars which are merely given for the purpose of information.

05-05-2025

| Patent document cited in search report | Publication date | Patent family member(s) | Publication date |
|---|---|---|---|
| WO 2014111856 A1 | 24-07-2014 | EP 2945610 A1 | 25-11-2015 |
| | | US 2014199254 A1 | 17-07-2014 |
| | | WO 2014111856 A1 | 24-07-2014 |
| US 2019343744 A1 | 14-11-2019 | NONE | |
| US 2005080135 A1 | 14-04-2005 | US 2005080135 A1 | 14-04-2005 |
| | | US 2005209327 A1 | 22-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003057182 A1
- US 5601811 A
- US 6426435 B
- US 6531628 B
- US 5427773 A
- US 6613340 B
- US 9463337 B2, Koshti